# EUROPEAN PATENT SPECIFICATION

(11) **EP 2 601 957 B1**
(45) Date of publication and mention of the grant of the patent: **10.04.2019**
(21) Application number: 11814313.0
(22) Date of filing: 05.08.2011
(51) Int. Cl.: A61K 35/14, A61P 31/00, A61P 37/04, C12N 5/00, C12N 5/078

(54) **IMMUNOLOGICAL FUNCTION ENHANCING AGENT**
WIRKSTOFF ZUR VERSTÄRKUNG IMMUNOLOGISCHER FUNKTIONEN
AGENT DESTINÉ À STIMULER LA FONCTION IMMUNITAIRE

(30) Priority: 06.08.2010 JP 2010177041
(43) Date of publication of application: 12.06.2013
(73) Proprietor: Canine-Lab. Inc., Koganei-shi, Tokyo 184-0012 (JP)
(72) Inventor: YAMAGUCHI, Tomohiro, Misato-shi Saitama 341-0018 (JP); OTSUKA, Hiromichi, Towadashi Aomori 034-0041 (JP)
(74) Representative: Abel & Imray
(86) International application number: PCT/JP2011/004455
(87) International publication number: WO 2012/017678

(56) References cited:
- EP-A2- 1 352 955
- US-A1- 2003 044 387
- US-A1- 2008 292 605
- OHTSUKA HIROMICHI ET AL: "Changes in peripheral leukocyte populations of weak calf syndrome of Japanese Black calves", JOURNAL OF VETERINARY MEDICAL SCIENCE - NIHON JUIGAKU ZASSHI, JAPANESE SOCIETY OF VETERINARY SCIENCE, TOKYO, JP, vol. 65, no. 7, 1 July 2003 (2003-07-01), pages 793-796, XP009174312, ISSN: 0916-7250
- IGIETSEME J U ET AL: "Quantitative measurement of T-lymphocyte activation by an enzyme-linked immunosorbent assay (ELISA) detecting interleukin-2 receptor expression", JOURNAL OF IMMUNOLOGICAL METHODS, ELSEVIER SCIENCE PUBLISHERS B.V.,AMSTERDAM, NL, vol. 97, no. 1, 26 February 1987 (1987-02-26), pages 123-131, XP023675651, ISSN: 0022-1759, DOI: 10.1016/0022-1759(87)90114-1 [retrieved on 1987-02-26]
- MASAHIRO OHIRA ET AL: "Adoptive immunotherapy with liver allograft-derived lymphocytes induces anti-HCV activity after liver transplantation in humans and humanized mice", JOURNAL OF CLINICAL INVESTIGATION, AMERICAN SOCIETY FOR CLINICAL INVESTIGATION, US , vol. 119, no. 11 2 November 2009 (2009-11-02), pages 3226-3235, XP002676751, ISSN: 0021-9738, DOI: 10.1172/JCI38374 Retrieved from the Internet: URL:http://www.jci.org/articles/view/38374 [retrieved on 2009-10-01]
- SLAVIN, S. ET AL.: 'Immunotherapy for resistant hematologic malignancies using matched or mismatched rIL-2 activated donor lymphocytes positively selected for CD56+ after allogeneic stem cell transplantation for allogeneic cell therapy without GVHD' BLOOD vol. 102, no. 11, 2003, page 400B, XP009175363
- TAKASU, M. ET AL.: 'Thymic hypoplasia in Japanese black calves with stillbirth/perinatal weak calf syndrome' JOURNAL OF VETERINARY MEDICAL SCIENCE vol. 70, no. 11, 2008, pages 1173 - 7, XP055098233
- OHTSUKA, H. ET AL.: 'Changes in peripheral leukocyte populations of weak calf syndrome of Japanese Black calves' JOURNAL OF VETERINARY MEDICAL SCIENCE vol. 65, no. 7, 2003, pages 793 - 796, XP009174312

## Description

### Technical Field

The present invention relates to an immune function enhancing agent (immunopotentiator). More specifically, the present invention relates to an immune function enhancing agent (immunopotentiator) of mammals other than human and a therapeutic agent for weak calf syndrome of mammals other than human, the agent containing allogeneic activated lymphocytes, and a method for producing these agents.

### Background Art

Herbivorous animals as typified by cattle and horses cannot receive immune substances such as antibodies and immune cells from their dams through the placenta in the fetal period. Therefore, neonates of these animals have poor immune function and may be affected by weak calf syndrome . The neonates of these animals commonly take immune substances such as antibodies and immune cells through colostrum, and thus the function of immune cells of the neonates is promoted. However, neonates affected by weak calf syndrome have low absorption of colostrum, thereby being easily affected by infectious diseases, and further, severe cases may lead to death. In addition, because there exist infectious diseases, which are infected through colostrum and cause serious conditions, in domestic animals, raw colostrum is not directly given from dam to neonate in production sites for the purpose of prevention of infections. There are not a few cases in which processed colostrum, which does not contain immune cells and whose immunity is low, is provided.

Activated lymphocyte therapy is known as one of cancer immunotherapy for small animals including human, dogs and cats . The activated lymphocyte therapy is a therapy in which autologous peripheral blood lymphocytes are activated and amplified in vitro and returned to the own body.

A formulation containing activated lymphocytes derived from another individual with the identical HLA (Human Leukocyte Antigen) is disclosed in JP 2004-2312 A (Patent Literature 1) . Because the formulation contains activated lymphocytes, it is believed to be effective in the prevention and treatment of autoimmune diseases.

An agent for enhancing the immune function of animals other than human and a therapeutic agent for weak calf syndrome have not, however, been developed.

### Citation List

### Patent Literature

Patent Literature 1: JP 2004-2312 A

EP1352922 discloses HLA matching donor-originating activated lymphocytes to be used in the prevention/treatment of tumors, infectious diseases and autoimmune diseases.

US 2003/0044387 A1 discloses activated lymphocyte preparations for preventing recurrence of carcinoma.

US2008/0292605 A1 discloses materials for stimulation of hematopoiesis by ex vivo activated cells.

### Non-Patent Literature

Blood, 2003, Vol. 102, No. 11, p. 400b (Slavin, S. et al) discloses immunotherapy for resistant hematologic malignancies using matched or mismatched rIL-2 activated donor lymphocytes positively selected for CD56+ after allogeneic cell therapy without GVHD.

Journal of Veterinary Medical Science, 2008, Vol. 70, No. 11, p.1173-7 (Takasu, M. et al) discloses thymic hypoplasia in black calves with stillbirth/perinatal weak calf syndrome.

Journal of Veterinary Medical Science, 2003, Vol. 65, No. 7, p. 793-796 (Ohtsuka, H. et al) discloses changes in peripheral leukocyte populations of weak calf syndrome of Japanese Black calves.

Journal of Immunological Methods, 1987, Vol. 97, No. 1, p. 123-131 (Igietseme, J. U. et al) discloses quantitative measurement of T-lymphocyte activation by an enzyme-linked immunosorbent assay (ELISA) detecting interleukin-2 receptor expression.

Journal of Clinical Investigation, 2009, Vol. 119, No. 11, p. 3226-3235 (Masahiro, O. et al) discloses adoptive immunotherapy with liver allograft-derived lymphocytes induces anti-HCV activity after liver transplantation in humans and humanized mice.

### Summary of Invention

### Technical Problem

Rare branded cattle such as Japanese black cattle and thoroughbreds with a good bloodline, for example, have a high likelihood of being affected by weak calf syndrome. Therefore, there is a high mortality rate among branded cattle and thoroughbreds in the neonatal period. In the meantime, when a component derived from an allogeneic animal is administered to them, serious side effects can occur.

Thus, an object of the present invention is to provide an immune function enhancing agent of nonautologous mammals other than human, which agent has few side effects. The immune function enhancing agent is effective as an agent for treating weak calf syndrome of neonatal animals

### Solution to Problem

The present invention is basically based on observation of examples in which in mammals other than human, particularly animals in which antibodies and immune cells do not transfer from mother to fetus through the placenta, even when using nonautologous activated lymphocytes, surprisingly the immune function of allogeneic animals can be enhanced without serious side effects and the lymphocytes are effective in treating particularly weak calf syndrome of neonatal animals.

The present invention relates to an immune function enhancing agent for allogeneic animals that contains activated lymphocytes derived from tissues of mammals other than human as an effective ingredient. The allogeneic animals are different from the animal from which the tissues derive but belong to the same species from which the tissues derive.

Activated lymphocytes derived from tissues of mammals other than human are activated lymphocytes which are activated by either one of interleukin-2 and anti-CD3 antibody or both.

In the present invention, the mammals are animals in which antibodies and immune cells do not transfer from mother to fetus through the placenta, in a specific
preferred embodiment cattle or horses.

As a preferred embodiment of the agent of the present invention, the agent is used as an agent for treating weak calf syndrome of neonates of animals in which antibodies and immune cells do not transfer from mother to fetus through the placenta. In the case of cattle or horses, even when allogeneic activated lymphocytes are administered to them, serious side effects are not confirmed. In addition, as described above, cattle or horses in the neonatal period, which are born with a weak constitution, are easily affected by infectious diseases such as diarrhea and pneumonia, and have a high likelihood of death, with no effect of treatment. By using the agent of the present invention, particularly weak calf syndrome of cattle or horses can be treated. Consequently, for example, cattle or horse neonates can be protected against infectious diseases.

The present invention also relates to a method for producing the immune function enhancing agent of allogeneic animals. The allogeneic animals are different from the animal from which the tissues derive but belong to the same species from which the tissues derive. This method includes the step of obtaining activated lymphocytes by amplifying and activating lymphocytes isolated
from mammals other than human, and the step of producing an agent using the obtained activated lymphocytes.

The step of obtaining activated lymphocytes includes a step in which lymphocytes are cultured in the presence of either one of interleukin-2 and anti-CD3 antibody or both.

An immune function enhancing agent for allogeneic animals is a therapeutic agent to treat weak calf syndrome of allogeneic neonates, which are the same species as mammals other than human. The mammals are animals in which antibodies and immune cells do not transfer from mother to fetus through the placenta.

Also disclosed herein is a method for enhancing the immune function of mammals other than human, particularly animals in which antibodies and immune cells do not transfer from mother to fetus through the placenta. This method includes the step of administering lymphocytes, which are isolated from an animal and amplified and activated, to allogeneic animals. The allogeneic animals are different from the animal from which the tissues derive but belong to the same species from which the tissues derive.

Also disclosed herein are methods in which the amplified and activated lymphocytes are administered to mammals other than human, particularly neonates of animals in which antibodies and immune cells do not transfer from mother to fetus through the placenta. Such methods are to enhance the immune function of neonates to treat weak calf syndrome.

Also disclosed herein are methods in which amplified and activated lymphocytes are administered to neonates, which are one year or less immediately after their birth and more preferably 3 months or less immediately after their birth, of animals in which antibodies and immune cells do not transfer from mother to fetus through the placenta. Animals under a few months after their birth are in the state of high immunological tolerance, and thus even when allogeneic activated lymphocytes are administered to the animals, immunological rejection rarely occurs. Therefore, the agent of the present invention can be used effectively to enhance the immune function of animals that are a few months of age.

### Advantageous Effects of Invention

According to the present invention, as proved by examples, there is provided, for animals in which antibodies and immune cells do not transfer from mother to fetus through the placenta, an immune function enhancing agent of allogeneic animals, which agent has few side effects. The immune function enhancing agent is effective as an agent for treating particularly weak calf syndrome of neonatal animals. In addition, according to the present disclosure, there is disclosed a method for producing such agent.

Further, there is disclosed a method for enhancing the immune function of animals in which antibodies and immune cells do not transfer from mother to fetus through the placenta. Therefore, high grade animals such as branded cattle and thoroughbreds can be efficiently raised.

### Brief Description of Drawings

Fig. 1 is a graph showing the growth curve of activated lymphocytes.
Fig. 2 is a graph showing changes of peripheral blood mononuclear cells (PBMC).
Fig. 3 is a graph showing changes of CD3-positive T lymphocytes.
Fig. 4 is a graph showing changes of B lymphocytes.
Fig. 5 is a graph showing changes of IgM B lymphocytes.
Fig. 6 is a graph showing changes of natural killer cells (NK cells) .
Fig. 7A is a graph showing changes of CD4⁺ cells. Fig. 7B is a graph showing changes of CD4⁺CD45R⁻ cells.
Fig. 8A is a graph showing changes of CD8⁺ cells. Fig. 8B is a graph showing changes of CD8⁺CD45R⁻ cells.
Fig. 9A, Fig. 9B, Fig. 9C and Fig. 9D are graphs showing the expression levels of IL-2 gene, IL-4 gene, IL-6 gene and IFN-γ gene, respectively.

### Description of Embodiments

The present invention relates to an immune function enhancing agent, which contains activated lymphocytes derived from tissues of mammals other than human as an effective ingredient, of animals which are the same species as the mammals and are different individuals from the mammals. The animals which are different individuals from the mammals are the same species as the mammals and mean so-called allogeneic. For example, when a subject from which lymphocytes are collected is a horse, the animals which are the same species as the mammals mean horses. Allogeneic animals mean nonautologous animals. An example of allogeneic animals is a mother and a child (a subject from which lymphocytes are collected is a dam and a subject to which an agent containing activated lymphocytes is administered is a calf) . Another example of allogeneic animals is a father and a child. Nevertheless, when allogeneic components are introduced into the animals, serious side effects are not confirmed as proved by examples. Therefore, the allogeneic animals may be animals which are the same species without a blood relationship.

In mammals, there are not only human, in which enough antibodies transfer through the placenta, but also animals in which antibodies and immune cells do not transfer from mother to fetus through the placenta. Examples of animals, in which antibodies and immune cells do not transfer from mother to fetus through the placenta, include cattle, horses, sheep, goats, pigs and pandas. The agent of the present invention is effectively used for these animals in which antibodies and immune cells do not transfer from mother to fetus through the placenta (hereinafter also referred to as "subject animals") . In the subject animals, immune substances including antibodies and immune cells cannot transfer from dam to fetus through the placenta at all in the fetus period. Among these animals, the agent of the present invention can be preferably used for cattle or horses.

Cattle has a high prevalence of weak calf syndrome (WCS) which occurs in neonatal calves. WCS is caused by a decrease in immune function (in particular, the function of T lymphocytes of immune cells) after birth of cattle. Therefore, the agent of the present invention is effective in treating particularly weak calf syndrome of cattle. The neonates of branded cattle have a high likelihood of death by infectious diseases. As shown in an example described below, the immune function of neonatal calves can be enhanced by the agent of the present invention, and thus the agent of the present invention can be effectively used for preventing infectious diseases of neonatal calves. As is the case with cattle, immune substances cannot transfer from a dam through the placenta in horses. Therefore, the agent of the present invention is effective in treating weak calf syndrome of horses.

As activated lymphocytes derived from tissues of animals in which antibodies and immune cells do not transfer from mother to fetus through the placenta, for example, lymphocytes which are isolated from peripheral blood of an animal and cultured can be used. Examples of tissues of animals include cells which exist in bone marrow such as a bone marrow stem cell. The activated lymphocytes are activated lymphocytes which are activated by interleukin-2 or anti-CD3 antibody.

When the activated lymphocytes are administered to cattle or horses, the present invention is effective in terms of being capable of administering allogeneic activated lymphocytes whose leukocyte antigen is not identical to the autologous one. In general, a probability that leukocyte antigens between individuals are identical is low. In addition, serious side effects occur by administering allogeneic activated lymphocytes with unidentical leukocyte antigen. Therefore, autologous activated lymphocytes are often used to treat infectious diseases using activated lymphocytes. Only when lymphocytes whose leukocyte antigen is identical to the autologous one can be found, allogeneic activated lymphocytes whose leukocyte antigen is identical to the autologous one have been used.

When autologous activated lymphocytes are used, it is necessary that blood be collected from autologous peripheral blood, lymphocytes isolated from the blood be activated, and the lymphocytes be cultured for about two weeks to amplify. Therefore, it has been impossible to prevent infectious diseases of neonates by administering autologous activated lymphocytes to neonates of cattle and horses, which have a high prevalence of weak calf syndrome (WCS), immediately after their birth.

When activated lymphocytes whose leukocyte antigen is identical to the autologous one are used, because a probability that leukocyte antigens between individuals are identical is low, it has been difficult to find an individual whose leukocyte antigen is identical to the autologous one only among livestock animals of branded cattle and thoroughbred horses which are raised. Further, when activated lymphocytes whose leukocyte antigen is identical to the autologous one are used, there have been problems that time and cost are required for an examination to confirm that an allogeneic leukocyte antigen is identical to the autologous leukocyte antigen.

When activated lymphocytes according to the present invention are administered to
cattle or horses, allogeneic activated lymphocytes whose leukocyte antigen is not identical to their leukocyte antigens can be administered to them. Therefore, a formulation of activated lymphocytes can be administered to neonates immediately after their birth by producing the formulation of allogeneic activated lymphocytes beforehand and storing it. Thus, the formulation of activated lymphocytes can prevent infectious diseases of neonates, and effectively act on treatment of particularly weak calf syndrome, which has been a problem. Further, when activated lymphocytes according to the present invention are administered to cattle or horses, because allogeneic activated lymphocytes whose leukocyte antigen is not identical to their leukocyte antigens can be administered to them, the step of examining leukocyte antigens can be eliminated. In addition, because leukocyte antigens are not required to be identical, a formulation of activated lymphocytes can be mass-produced by producing an activated lymphocyte of an individual in large quantities, and production costs can be reduced.

The method for producing an immune function enhancing agent of allogeneic animals, will be now described. This method includes the step of obtaining activated lymphocytes by amplifying and activating
lymphocytes isolated from animals in which antibodies and immune cells do not transfer from mother to fetus through the placenta, and the step of preparing the agent using the obtained activated lymphocytes. As the method for producing the activated lymphocytes, for example, a method in which a method disclosed in JP 2004-2312 A is suitably modified can be adopted. A preferred embodiment of the method is to include the step of culturing the isolated lymphocytes in the presence of interleukin-2 or a factor to promote the proliferation of T cells (e.g. anti-CD3 antibody). As proved by an example described below, by using IgG1 type as anti-CD3 antibody, activated lymphocytes can be effectively activated.

In the step of obtaining activated lymphocytes which are administered to cattle or horses, gelatin can be added to a culturing medium of lymphocytes. Gelatin promotes activation of lymphocytes . In addition, when gelatin is added to a medium, gelatin acts to protect activated lymphocytes. Further, as described below, because formulations containing activated lymphocytes which are administered to cattle or horses can be stored in a freezer, gelatin can function as a cushioning material during preservation by freezing.

As gelatin, pig skin gelatin, bovine bone gelatin, fish bone or fish skin gelatin can be used. To 100 parts by weight of a culturing medium, 0.1 to 10 parts by weight of gelatin can be added, preferably 1 to 5 parts by weight. Gelatin is mixed with a medium by a general method. When lymphocytes are cultured in a medium containing gelatin, culturing temperature can be 37°C to 42°C and it is preferred that culturing be carried out in a relatively high temperature range of 39°C to 42°C. A temperature from 39°C to 42°C is higher than general culturing temperature. A temperature from 39°C to 42°C is, however, a temperature at which gelatin can be uniformly dissolved and which is close to average body temperature of cattle or horses. Therefore, by culturing lymphocytes which are administered to cattle or horses at a temperature from 39°C to 42°C, lymphocytes which are more active in cattle or horses can be obtained.

The enhancing agent of the present invention can be stored in a freezer after produced, and for example, when a neonate is born, the agent can be thawed and administered. The agent of the present invention can be prepared as a liquid medicine and provided as a kit in which the liquid medicine is frozen with male sperms of branded cattle and racehorses (thoroughbreds). As described above, by forming a kit using male sperms and an activated lymphocyte formulation, an activated lymphocyte formulation which is less prone to produce side effects can be sold. That is, because for a neonate born after fertilization using sperms contained in such kit, an agent exists, which contains activated lymphocytes derived from its farther, the agent of the present invention can be administered to the neonate, with fewer side effects. Seed bulls and studhorses are limited among branded cattle and thoroughbreds, and thus the kit of the present invention can be effectively used.

The dose of a formulation containing activated lymphocytes as a main component can be suitably varied depending on, for example, a type of target animal in which antibodies and immune cells do not transfer from mother to fetus through the placenta. An example of the dose of a formulation per once is not less than 1 × 10² and not more than 1 × 10⁹ lymphocytes per kg of body weight. The dose of a formulation per once can be not less than 1 × 10³ and not more than 1 × 10¹¹, and not less than 1 × 10⁴ and not more than 1 × 10¹⁰ lymphocytes.

For the agent of the present invention, a known dosage form can be adopted. The preferred dosage form of the agent of the present invention is an injectable solution. An example of the agent is an injectable solution in which the appropriate amount of activated lymphocytes is dispersed in a physiological salt solution containing 0.01 to 5% by volume of blood serum or serum albumin. An example of a method for administering the injectable solution is intravenous injection. An example of administration frequency is once per day or more and once per month or less.

Also disclosed herein is a method for enhancing the immune function of animals in which antibodies and immune cells do not transfer from mother to fetus through the placenta. The method includes the step of administering lymphocytes, which are isolated from an animal and amplified and activated, to allogeneic animals which are the same species as the animal.

Also disclosed herein are methods in which the amplified and activated lymphocytes are administered to neonates of animals in which antibodies and immune cells do not transfer from mother to fetus through the placenta. Such methods are to enhance the immune function of neonates to treat weak calf syndrome.

Also disclosed herein are methods in which amplified and activated lymphocytes are administered to neonates, which are one year or less immediately after their birth and more preferably 3 months or less immediately after their birth, of animals in which antibodies and immune cells do not transfer from mother to fetus through the placenta. Animals under a few months after their birth are in the state of high immunological tolerance, and thus even when allogeneic activated lymphocytes are administered to the animals, immunological rejection rarely occurs. Therefore, the agent of the present invention can be effectively used to enhance the immune function of animals that are a few months of age.

### Example 1

### Preparation of activated lymphocytes

In this example, lymphocytes were collected from bovine peripheral blood and the collected lymphocytes were activated using IL-2 and anti-bovine CD3 antibody to obtain activated lymphocytes. Each step will be now described.

### Preparation of a flask for culturing activated lymphocytes

Anti-bovine CD3 antibody (MM1A (IgG1), manufactured by VNRD) was diluted to a concentration of 2.5 µl/ml using sterilized phosphate buffered saline (PBS), and 10 ml of the obtained solution was poured into a flask with a surface area of 75 cm² (manufactured by SUMITOMO BAKELITE CO., LTD.). This antibody diluted solution was spread uniformly on the bottom of the flask, and the flask was left to stand in a refrigerator (4°C) until used and a solid phase was obtained. When used, the flask was taken out from the refrigerator, and the antibody diluted solution was removed by vacuum, and washing was then carried out three times using sterilized PBS. Ultimately, the remaining solution used for washing was sufficiently removed by vacuum to prepare a flask for culturing activated lymphocytes.

### Preparation of a culturing medium

To LAM-1 medium containing 700 U/ml of interleukin (IL)-2 (manufactured by Cenine-Lab. Inc.), bovine calf serum (manufactured by JRH Biosciences, radiation sterilized) was added so that the volume ratio would be 2.5% to prepare a medium. As LAM-2 medium containing 175 U/ml of IL-2 (manufactured by Cenine-Lab. Inc.) and LAM-3 medium containing 175 U/ml of IL-2 (in a gas permeable bag, total amount 750 ml, manufactured by Cenine-Lab. Inc.), which were used in the middle of the culturing step, commercially available products were used.

### Isolation of lymphocytes from peripheral blood

Using a heparin treated blood collection tube, 20 ml of peripheral whole blood was collected from the bovine jugular vein. The peripheral blood was centrifuged at 1,600 rpm for 10 minutes, and the isolated blood plasma was placed in a new sterile tube. To the centrifuged precipitate containing blood cell components, RPMI-1640 medium (manufactured by Wako Pure Chemical Industries, Ltd.) was added so that the gross amount would be 40 ml, which was twice the whole blood volume, to dilute, and 10 ml of the diluted blood was added to 3 ml of Ficoll-Paque (manufactured by GE Healthcare). The obtained mixture was centrifuged at 1,600 rpm at 22°C for 30 minutes to retrieve a peripheral blood mononuclear cell (PBMC) layer containing lymphocytes. The obtained layer was washed once with RPMI-1640 medium. By this operation, about 1 × 10⁷ peripheral blood mononuclear cells (PBMC) were obtained.

### Culturing lymphocytes

The retrieved PBMC was suspended in 20 ml of LAM-1 medium containing 700 U/ml of IL-2 and 2.5% of bovine calf serum by volume ratio, and the obtained suspension was placed in a flask with solid phased anti-bovine CD3 antibody, and culturing was carried out. On the third day of culturing, 20 ml of LAM-2 medium was added thereto, and after 2 days of that (the fifth day of culturing), 40 ml of LAM-2 medium was added thereto. After 2 days of that (the seventh day of culturing), sufficient proliferation of cells was confirmed, and the total amount of the cell suspension was moved to LAM-3 medium. Thereafter, for 5 to 10 days until amplified cells were retrieved, the flask was left to stand in a carbon dioxide incubator. The culturing temperature can be 37°C to 42°C and culturing can be preferably carried out in a relatively high temperature range of 39°C to 42°C

### Preparation of a cultured lymphocyte formulation

In the case of cultured lymphocytes which were decided to be administered, a culturing bag container (LAM-3) containing a cultured lymphocyte-suspended culture solution was sufficiently stirred, and a cell suspension solution was retrieved from a sample port to a centrifugal tube and centrifuged at 1600 rpm for 7 minutes to retrieve cultured lymphocytes. After that, the retrieved lymphocytes were washed twice with a physiological salt solution containing 0.1% of autoserum (blood plasma obtained by centrifugation from whole blood was stored in a refrigerator for 1 day or more, and then incubated at 56°C for 30 minutes to inactivate, and the blood plasma was further stored in a refrigerator for 1 day or more and centrifuged at 3,000 rpm for 20 minutes, and the obtained supernatant was used as an autoserum), and ultimately suspended in 50 ml of a physiological salt solution containing 1% autoserum. The suspension was filled in an injection syringe to obtain a formulation. In addition, the determination of endotoxin in the activated lymphocyte-suspended culture solution (Toxicolor test, manufactured by SEIKAGAKU CORPORATION) and cultivation of bacteria using Trypto-Soya Agar (manufactured by NISSUI) were carried out to confirm safety before administration.

### Determination of the surface type of cultured lymphocyte

In a test tube, 5 × 10⁵ cultured lymphocytes obtained in the above were retrieved and washed once by adding PBS and supernatant was then removed. For direct staining and indirect staining, 10 µl of a FITC (fluorescein isothiocyanate) or PE (phycoerythrin) labeled monoclonal antibody and 1 µg of an unlabeled monoclonal antibody, respectively, were added thereto and sufficiently stirred. The test tube was left to stand in a refrigerator for 30 minutes and washing was carried out once with PBS. In the case of indirect staining, 50 µl of FITC labeled sheep anti-mouse antibody (manufactured by AbD Serotec), suitably diluted, was added thereto and sufficiently stirred and the test tube was left to stand in a refrigerator for 30 minutes. After that, washing was carried out once with PBS, and ultimately 0.5 ml of a sheath solution was added thereto and sufficiently stirred. Surface antigens which had reacted with each antibody were determined by CyAn (manufactured by Dako) . Monoclonal antibodies used for determination were FITC labeled anti-bovine CD8 antibody (9ACT80C, manufactured by VNRD), PE labeled anti-bovine CD4 antibody (CACT183B, manufactured by VNRD) and PE labeled anti-canine CD21 antibody (MCA1781PE, manufactured by AbD Serotec) for direct staining, and anti-bovine CD3 antibody (MM1A, manufactured by VNRD), anti-bovine γδT cell antibody (WC-1, manufactured by AbD Serotec) and anti B-B7 (CD21-like) antibody (GB25A, manufactured by VMRD, Inc.) for indirect staining. For determining non-specific reactions as control, FITC labeled anti-mouse IgG1 antibody (manufactured by Dako) and PE labeled anti-mouse IgG1 antibody (manufactured by Dako) were used. The results are shown in Table 1.

**[Table 1]**

| Surface Type of Cultured Lymphocytes (%) | | |
|---|---|---|
| Surface type | After separation | The 14th day of culture |
| CD3 | 65.34 | 99.41 |
| CD4 | 25.29 | 13.60 |
| CD8 | 21.34 | 78.98 |
| CD21 | 16.76 | 0.30 |
| *γ δ*T | 15.21 | 1.17 |

### Example 2

In this example, activated lymphocytes derived from a dam were obtained and an agent containing the activated lymphocytes was administered to neonatal calves. Each step in this example will be now described.

### Preparation of activated lymphocytes

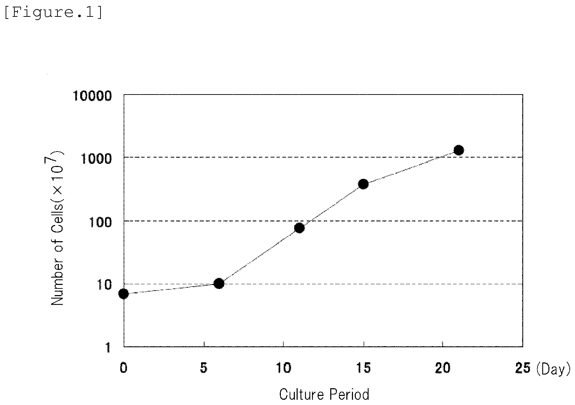

Peripheral whole blood was collected from dams of Holstein strain, Japanese Black strain and a hybrid strain before 2 to 3 weeks of the expected delivery date. According to Example 1, activated lymphocytes were continuously cultured until the delivery date. Fig. 1 is a graph showing the growth curve of activated lymphocytes.

### Administration of activated lymphocytes to calves

The cultured lymphocytes were retrieved within 2 days after the birth dates of neonatal calves (0 days old), and introduced through the jugular veins of the neonatal calves. The dose was about 1 × 10⁹ activated lymphocytes. These neonatal calves were considered as an activated lymphocyte administered group. Meanwhile, among neonatal calves, a group to which activated lymphocytes were not administered was considered as a control group. To remove influence by intake of colostrum from dams, commercially available powdered colostrum formulations were provided for all neonatal calves after their birth. After that, the first vaccination at two weeks old and the second vaccination at 6 weeks old were carried out, and peripheral blood was collected eight times in total, at 0, 3 and 7 days old, 2 weeks old and after 3 days of that, 6 weeks old and after 3 days and 6 days of that. A fraction of leukocytes in the peripheral blood was analyzed and the surface type of lymphocyte was determined, and the expression levels of cytokine genes by the PHA (phytohemagglutinin) stimulus were analyzed.

### Determination of the surface type of lymphocyte in peripheral blood

The surface type of lymphocyte in peripheral blood was determined in the same manner as in Example 1.

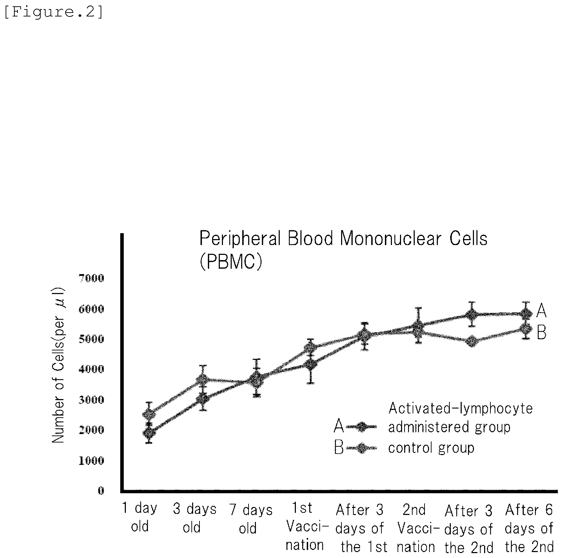

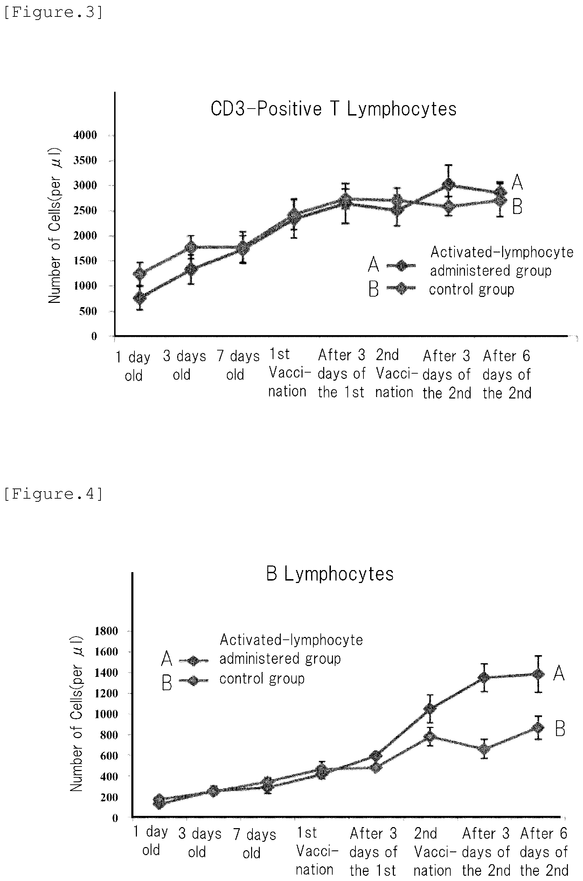

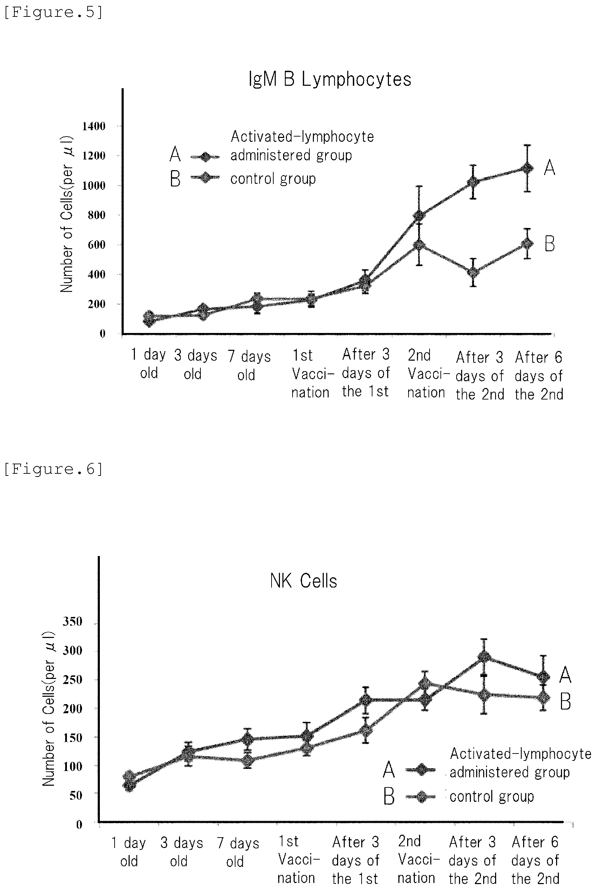

Fig. 2 is a graph showing changes of peripheral blood mononuclear cells (PBMC). Fig. 3 is a graph showing changes of CD3-positive T lymphocytes. Fig. 4 is a graph showing changes of B lymphocytes. Fig. 5 is a graph showing changes of IgM B lymphocytes. Fig. 6 is a graph showing changes of natural killer cells (NK cells).

As can be seen from Fig. 2, Fig. 3 and Fig. 6, in both the activated lymphocyte administered group and the control group, the number of peripheral blood mononuclear cells (PBMC), the number of CD3-positive T lymphocytes, the number of CD8 killer T cells and the number of NK cells were slowly increased after birth. It is found that, however, cell proliferation in the activated lymphocyte administered group was high as compared to that in the control group after the second vaccination.

Meanwhile, as can be seen from Fig. 4 and Fig. 5, B lymphocytes (MHC class-II⁺CD14⁻ B cells) and IgM B lymphocytes (CD21⁺IgM⁺ B cells) in the activated lymphocyte administered group were significantly increased as compared to those in the control group after 3 days and 6 days of the second vaccination.

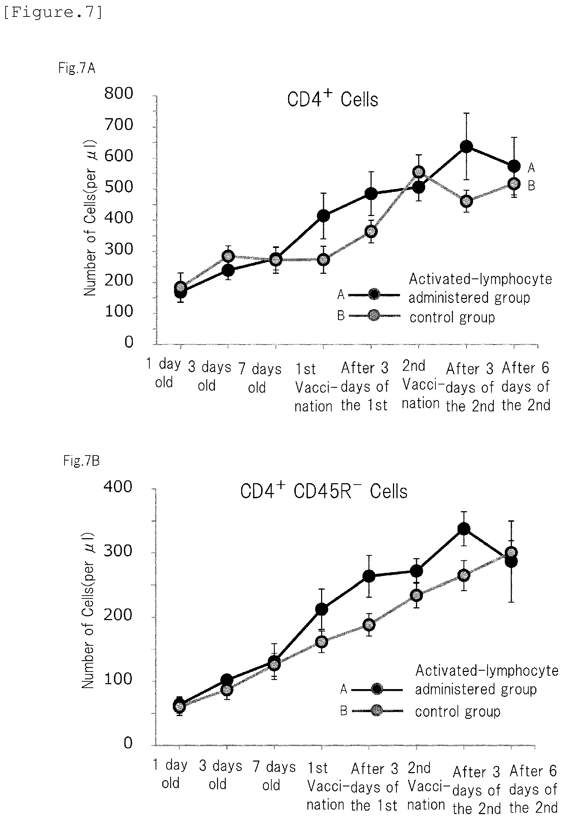

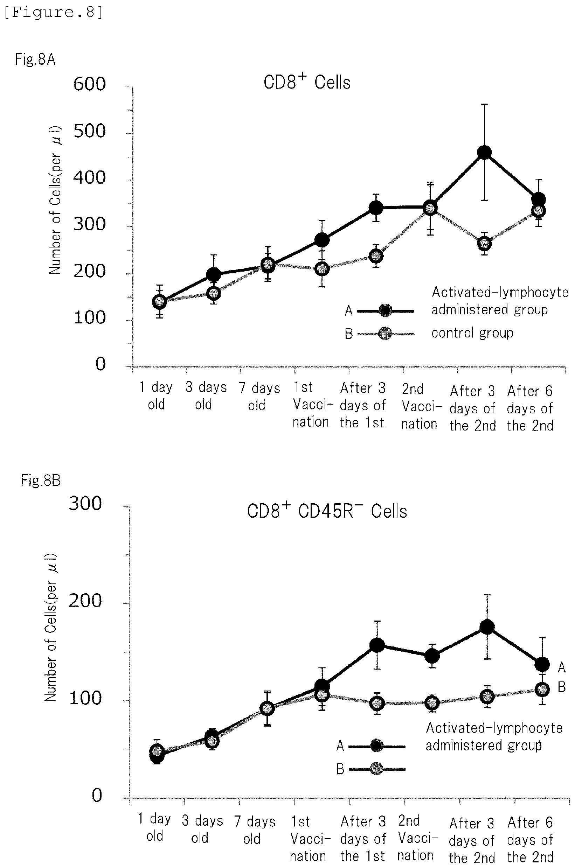

Fig. 7A is a graph showing changes of CD4⁺ cells. Fig. 7B is a graph showing changes of CD4⁺CD45R⁻ cells. Fig. 8A is a graph showing changes of CD8⁺ cells. Fig. 8B is a graph showing changes of CD8⁺CD45R⁻ cells. As can be seen from Fig. 7A, Fig. 7B, Fig. 8A and Fig. 8B, cell proliferation in the activated lymphocyte administered group was high as compared to that in the control group. This shows that the immune function of neonatal calves was enhanced by the activated lymphocytes in the present example. In addition, these cells were slowly increased after the first vaccination, and further increased after the second vaccination. This shows that the antigen responsiveness of the cells was increased by the first vaccination and responsiveness to the antigens was further increased at the second vaccination.

### Analysis of the expression levels of cytokine genes

Peripheral blood mononuclear cells (PBMC) were isolated from heparin added blood using a density medium and mRNA was extracted. By reference to a conventional method, cDNA was synthesized using the extracted mRNA and real-time PCR was carried out. β-Actin was used as an internal standard gene. The real-time PCR was carried out by SYBR Green PCR Master Mix (Applied Biosystems, CA, USA) using 7700 Sequence Detector according to a reported method.

PCR conditions were in accordance with the operation manual of Step One Plus™ Real-Time PCR System (Applied Biosystem Japan) . A sample was set to the system and the system was performed at 50°C for 30 minutes and at 95°C for 15 minutes once each, and a reaction cycle at 95°C for 15 minutes and at 60°C for 1 minute was repeated 45 times.

Target genes, and forward primers and reverse primers for PCR were as shown in Table 2 below.

**[Table 2]**

| Target Genes | Forward Primers | Reverse Primers |
|---|---|---|
| IL-4 | TGCCCCAAAGAACACAACTG | TTTAGCCTTTCCAAGAGGTC |
| IL-6 | TGAAAGCAGCAAGGAGACAC | TGACATTTTCCTGATTTCCC |
| IL-12 | AGGTCGTGGTAGAAGCTGTG | CCTTGTGGCATGTGACTTTG |
| IFN-γ | AGCCCAGATGTAGCTAAGGG | CTCCAGTTTCTCAGAGCTGC |
| *β*-actin | CTTTTACAACGAGCTGCGTG | CACGGTCCGTGAGGATCTTC |

The expression levels of cytokine genes were determined using the Threshold Cycle (Ct value) of each amplified gene by the formula: the expression level of cytokine gene = 2^{(-(Ct value of cytokine/Ct value of b-actin))}.

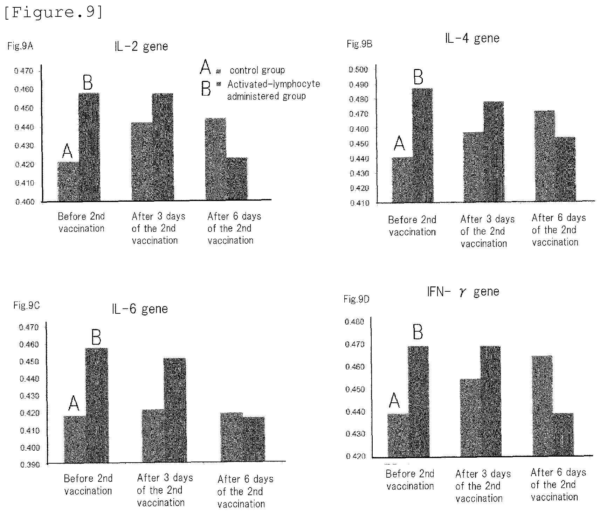

The results are shown in Fig. 9. Figs. 9A, B, C and D are graphs showing the expression levels of IL-2 gene, IL-4 gene, IL-6 gene and IFN-γ gene, respectively. As can be seen from Figs. 9A, B, C and D, about all cytokine genes, the expression levels of cytokines in the activated lymphocyte administered group were high as compared to those in the control group before and after 3 days of the second vaccination, but the expression levels of cytokines in the activated lymphocyte administered group were low as compared to those in the control group after 6 days. This shows that the immune function of neonatal calves was enhanced by activated lymphocytes in the present example at an early time.

In the example described above, although an example of a neonate and a real mother as allogeneic animals which are nonautologous was used, an example of a birth by embryo transfer to a cow other than a real mother was contained. The present invention is effective for an allogeneic relationship which does not have a mother child relationship.

### Example 3

In this example, activated lymphocytes derived from a mother horse were obtained and an agent containing the activated lymphocytes was administered to neonatal horses.

Activated lymphocytes were prepared in the same manner as in Example 2 except that lymphocytes were collected using peripheral blood derived from mother horses and the collected lymphocytes were activated using IL-2 and an anti-horse antibody. An agent containing the activated lymphocytes was administered to neonatal horses in the same manner as in Example 2. The neonatal horses were considered as an activated lymphocyte administered group. Meanwhile, among neonatal horses, a group to which activated lymphocytes were not administered was considered as a control group. To remove influence by intake of colostrum from mother horses, commercially available powdered colostrum formulations were provided for all neonatal horses after their birth. After that, the first vaccination at two weeks old and the second vaccination at 6 weeks old were carried out, and peripheral blood was collected eight times in total, at 0, 3 and 7 days old, 2 weeks old and after 3 days of that, 6 weeks old and after 3 days and 6 days of that. A fraction of leukocytes in the peripheral blood was analyzed and the surface type of lymphocyte was determined, and the expression levels of cytokine genes by the PHA (phytohemagglutinin) stimulus were analyzed.

The results showed that the number of T cells and the number of NK cells in peripheral blood of the activated lymphocyte administered group were increased as compared to those of the control group from the third day to the seventh day after vaccination, and further showed that the expression levels of cytokine genes in the activated lymphocyte administered group were increased as compared to those in the control group. This showed that the immune function of neonatal horses was enhanced at an early time like cattle by administering activated lymphocytes derived from mother horses to the neonatal horses.

### Industrial Applicability

The agent of the present invention and the method for producing the same can be utilized in the pharmaceutical industry as an immune function enhancing agent of animals, in which antibodies and immune cells do not transfer from mother to fetus through the placenta, and a therapeutic agent for weak calf syndrome. In addition, a method for enhancing the immune function of animals in the present invention can be used in the veterinary field.

### Sequence Listing Free Text

SEQ ID NO:1: primer
SEQ ID NO:2: primer
SEQ ID NO:3: primer
SEQ ID NO:4: primer
SEQ ID NO:5: primer
SEQ ID NO:6: primer
SEQ ID NO:7: primer
SEQ ID NO:8: primer
SEQ ID NO:9: primer
SEQ ID NO:10: primer

## Claims

1. An immune function enhancing agent for use in the prevention and/or treatment of weak calf syndrome in neonates of allogeneic animals,
wherein the agent comprises activated lymphocytes derived from tissues of mammal other than human as an effective ingredient, **characterized in that**, the allogeneic animals are different from the mammal from which the tissues derive,
wherein the allogeneic animals are animals whose leukocyte antigen is not identical to a leukocyte antigen of the mammal from which the tissues derive, and
wherein the mammal is an animal in which antibodies and immune cells do not transfer from a mother to a fetus through the placenta,
wherein the activated lymphocytes are activated by either one or both of interleukin-2 and anti-CD3 antibody.

2. An agent for use in accordance with claim 1,
wherein the mammal is a cattle, a horse, a sheep, a goat, a pig or a panda.

3. An agent for use in accordance with claim 1 or 2,
wherein the mammal is a cattle or a horse.

4. An agent for use in accordance with claim 1,
wherein the immune function enhancing agent is an agent for use in treating weak calf syndrome of neonates of mammals.

5. An agent for use in accordance with claim 1,
wherein the weak calf syndrome comprises the weak calf syndrome derived from infections.

6. An agent for use in accordance with claim 1, wherein the agent is produced by a method comprising steps of:
obtaining activated lymphocytes by amplifying and activating lymphocytes isolated from a mammal other than human; and
producing the immune function enhancing agent using the activated lymphocytes.

7. An agent for use in accordance with claim 6, wherein the mammals are cattle, horses, sheep, goats, pigs or pandas.

8. An agent for use in accordance with claim 6 or 7,
wherein the step of obtaining activated lymphocytes comprises a process of cultivating activated lymphocytes in the presence of either one or both of interleukin-2 and anti-CD3 antibody.

9. An agent for use in accordance with claim 6 or 7, wherein the mammal is a cattle,
wherein the step of obtaining activated lymphocytes comprises a process of cultivating activated lymphocytes in the presence of anti-CD3 antibody, and
wherein the anti-CD3 antibody is IgG1 type antibody.

10. An agent for use in accordance with claim 6 or 7,
wherein the immune function enhancing agent of allogeneic animals is a therapeutic agent of weak calf syndrome to treat weak calf syndrome of allogeneic neonates.

## Patentansprüche

1. Ein Mittel zur Verstärkung der Immunfunktion zur Verwendung bei der Prävention und/oder Behandlung des Weak-Calf-Syndroms bei Neugeborenen von allogenen Tieren,
wobei das Mittel als wirksamen Inhaltsstoff aktivierte Lymphozyten umfasst, die von Geweben eines Säugers stammen, der kein Mensch ist,
**dadurch gekennzeichnet, dass** die allogenen Tiere sich vom Säuger, von dem die Gewebe stammen, unterscheiden,
wobei die allogenen Tiere Tiere sind, deren Leukozytenantigen nicht mit einem Leukozytenantigen des Säugers, von dem die Gewebe stammen, identisch ist, und wobei der Säuger ein Tier ist, bei dem sich Antikörper und Immunzellen nicht von einer Mutter auf einen Fötus durch die Plazenta übertragen,
wobei die aktivierten Lymphozyten entweder durch eines von Interleukin-2 und Anti-CD3-Antikörper oder durch beide aktiviert werden.

2. Ein Mittel zur Verwendung gemäß Anspruch 1, wobei der Säuger ein Rind, ein Pferd, ein Schaf, eine Ziege, ein Schwein oder ein Panda ist.

3. Ein Mittel zur Verwendung gemäß Anspruch 1 oder 2, wobei der Säuger ein Rind oder ein Pferd ist.

4. Ein Mittel zur Verwendung gemäß Anspruch 1, wobei das Mittel zur Verstärkung der Immunfunktion ein Mittel zur Verwendung bei der Behandlung des Weak-Calf-Syndroms bei Neugeborenen von Säugern ist.

5. Ein Mittel zur Verwendung gemäß Anspruch 1, wobei das Weak-Calf-Syndrom das von Infektionen herrührende Weak-Calf-Syndrom umfasst.

6. Ein Mittel zur Verwendung gemäß Anspruch 1, wobei das Mittel durch ein Verfahren hergestellt wird, umfassend die Schritte:
Gewinnen von aktivierten Lymphozyten durch Verstärken und Aktivieren von Lymphozyten, die von einem Säuger, der kein Mensch ist, isoliert wurden, und
Erzeugen des Mittels zur Verstärkung der Immunfunktion unter Verwendung der aktivierten Lymphozyten.

7. Ein Mittel zur Verwendung gemäß Anspruch 6, wobei die Säuger Rinder, Pferde, Schafe, Ziegen, Schweine oder Pandas sind.

8. Ein Mittel zur Verwendung gemäß Anspruch 6 oder 7, wobei der Schritt des Gewinnens von aktivierten Lymphozyten ein Verfahren zur Kultivierung aktivierter Lymphozyten in Gegenwart von entweder einem von Interleukin-2 und Anti-CD3-Antikörper oder beiden umfasst.

9. Ein Mittel zur Verwendung gemäß Anspruch 6 oder 7,
wobei der Säuger ein Rind ist,
wobei der Schritt des Gewinnens von aktivierten Lymphozyten ein Verfahren zur Kultivierung von aktivierten Lymphozyten in Gegenwart von Anti-CD3-Antikörper umfasst, und
wobei der Anti-CD3-Antikörper ein Antikörper vom Typ IgG1 ist.

10. Ein Mittel zur Verwendung gemäß Anspruch 6 oder 7, wobei das Mittel zur Verstärkung der Immunfunktion von allogenen Tieren ein therapeutisches Mittel für das Weak-Calf-Syndrom zur Behandlung des Weak-Calf-Syndroms von allogenen Neugeborenen ist.

## Revendications

1. Agent stimulant la fonction immunitaire destiné à être utilisé dans la prévention et/ou le traitement du syndrome du veau faible chez des nouveau-nés d'animaux allogéniques,
où l'agent comprend des lymphocytes activés dérivés de tissus d'un mammifère autre qu'un humain en tant qu'ingrédient efficace, **caractérisé en ce que** les animaux allogéniques sont différents du mammifère à partir duquel le tissu est dérivé,
où les animaux allogéniques sont des animaux dont l'antigène des leucocytes n'est pas identique à un antigène des leucocytes du mammifère à partir duquel le tissu est dérivé, et
où le mammifère est un animal chez qui les anticorps et les cellules immunitaires ne sont pas transférés d'une mère à un foetus par le placenta,
où les lymphocytes activés sont activés par l'interleukine-2 et/ou un anticorps anti-CD3.

2. Agent destiné à être utilisé selon la revendication 1, où le mammifère est un bovin, un cheval, un mouton, une chèvre, un porc ou un panda.

3. Agent destiné à être utilisé selon la revendication 1 ou 2, où le mammifère est un bovin ou un cheval.

4. Agent destiné à être utilisé selon la revendication 1, où l'agent stimulant la fonction immunitaire est un agent destiné à être utilisé dans le traitement du syndrome du veau faible chez des nouveau-nés de mammifères.

5. Agent destiné à être utilisé selon la revendication 1, où le syndrome du veau faible comprend le syndrome du veau faible dérivé d'infections.

6. Agent destiné à être utilisé selon la revendication 1, où l'agent est produit par un procédé comprenant les étapes consistant à :
obtenir des lymphocytes activés en amplifiant et en activant des lymphocytes isolés à partir d'un mammifère autre qu'un humain ; et
produire l'agent stimulant la fonction immunitaire en utilisant les lymphocytes activés.

7. Agent destiné à être utilisé selon la revendication 6, où les mammifères sont des bovins, des chevaux, des moutons, des chèvres, des porcs ou des pandas.

8. Agent destiné à être utilisé selon la revendication 6 ou 7, où l'étape consistant à obtenir des lymphocytes activés comprend un procédé de culture de lymphocytes activés en présence de l'interleukine-2 et/ou d'un anticorps anti-CD3.

9. Agent destiné à être utilisé selon la revendication 6 ou 7,
où le mammifère est un bovin,
où l'étape consistant à obtenir des lymphocytes activés comprend un procédé de culture de lymphocytes activés en présence d'un anticorps anti-CD3, et
où l'anticorps anti-CD3 est un anticorps de type IgG1.

10. Agent destiné à être utilisé selon la revendication 6 ou 7, où l'agent stimulant la fonction immunitaire d'animaux allogéniques est un agent thérapeutique du syndrome du veau faible pour traiter le syndrome du veau faible chez des nouveau-nés allogéniques.


### SEQUENCE LISTING

<110> Canine-Lab. Inc.,
<120> An activate agent for immuno function
<130> 10-144P
<160> 10
<170> PatentIn version 3.5
<210> 1
   <211> 20
   <212> DNA
   <213> Artificial Sequence
<220>
   <223> Primer
<400> 1
   tgccccaaag aacacaactg 20
<210> 2
   <211> 20
   <212> DNA
   <213> Artificial Sequence
<220>
   <223> Primer
<400> 2
   tttagccttt ccaagaggtc 20
<210> 3
   <211> 20
   <212> DNA
   <213> Artificial Sequence
<220>
   <223> Primer
<400> 3
   tgaaagcagc aaggagacac 20
<210> 4
   <211> 20
   <212> DNA
   <213> Artificial Sequence
<220>
   <223> Primer
<400> 4
   tgacattttc ctgatttccc 20
<210> 5
   <211> 20
   <212> DNA
   <213> Artificial Sequence
<220>
   <223> Primer
<400> 5
   aggtcgtggt agaagctgtg 20
<210> 6
   <211> 20
   <212> DNA
   <213> Artificial Sequence
<220>
   <223> Primer
<400> 6
   ccttgtggca tgtgactttg 20
<210> 7
   <211> 20
   <212> DNA
   <213> Artificial Sequence
<220>
   <223> Primer
<400> 7
   agcccagatg tagctaaggg 20
<210> 8
   <211> 20
   <212> DNA
   <213> Artificial Sequence
<220>
   <223> Primer
<400> 8
   ctccagtttc tcagagctgc 20
<210> 9
   <211> 20
   <212> DNA
   <213> Artificial Sequence
<220>
   <223> Primer
<400> 9
   cttttacaac gagctgcgtg 20
<210> 10
   <211> 20
   <212> DNA
   <213> Artificial Sequence
<220>
   <223> Primer
<400> 10
   cacggtccgt gaggatcttc 20